# FASCICULE DE BREVET EUROPEEN

(11) **EP 0 374 509 B1**
(45) Date de publication et mention de la délivrance du brevet: **05.01.1994**
(21) Numéro de dépôt: 89121633.5
(22) Date de dépôt: 23.11.1989
(51) Int. Cl.: C07D 303/04, C07C 47/32, C07C 31/135

(54) **4,4,5,8-Tétraméthyl-1-oxaspiro[2.5]octane, procédé pour sa préparation et son utilisation en tant que produit de départ pour la préparation du 2,2,3,6-tétraméthyl-cyclohexane-carbaldéhyde**
4,4,5,8-Tetramethyl-1-oxaspiro(2.5)octan, Verfahren zu seiner Herstellung und seine Verwendung als Ausgangsprodukt in der Herstellung von 2,2,3,6-Tetramethyl-cyclohexan-carboxaldehyd
4,4,5,8-Tetramethyl-1-oxaspiro(2.5) octane, process for its preparation and its use as a starting material in the preparation of 2,2,3,6-tetramethyl cyclohexane carboxaldehyde

(30) Priorité: 21.12.1988 CH 4759/88
(43) Date de publication de la demande: 27.06.1990
(73) Titulaire: FIRMENICH SA, CH-1211 Genève 8 (CH)
(72) Inventeur: Chapuis, Christian, CH-1209 Petit-Saconnex (CH); Margot, Christian, CH-1196 Gland (CH); Schulte-Elte, Karl-Heinrich, CH-1213 Onex (CH); Pamingle, Hervé, CH-1290 Versoix (CH)
(74) Mandataire: Salvadori, Giuseppe

(56) Documents cités:
- EP-A- 0 124 011
- GB-A- 1 478 764
- HELVETICA CHIMICA ACTA, vol. 68, no. 7, 13 novembre 1985, pages 1961-1985, Bâle, CH; K.H. SCHULTE-ELTE et al.: "Diastereoselektivität der Geruchswahrnehmung von Alkoholen der Iononreihe"
- Helv. Chim. Acta 63 (1980) p. 1665-1674

## Description

Le brevet européen n^{o} 121'828 publié le 19 novembre 1987 et portant les priorités du 12.04.83 et du 30.08.83, décrit des composés de formule 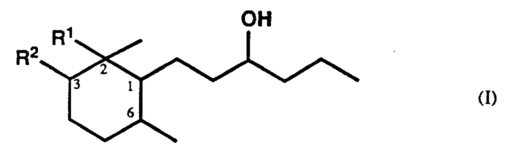
dans laquelle chacun des symboles R¹ et R² sert à désigner un radical méthyle ou dans laquelle R¹ désigne un radical éthyle et R² un radical méthyle ou l'hydrogène. Un des aspects particuliers du brevet cité a trait à l'utilisation, en tant qu'ingrédients parfumants, des composés (I) dans leur configuration trans de formule 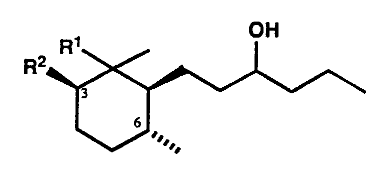
ou des mélanges contenant des quantités prépondérantes (supérieures à 50% en poids) desdits composés accompagnés par des quantités inférieures de leurs isomères cis correspondants.

Parmi les composés définis à l'aide de la formule (I), le 1-(2,2,3,6-tétraméthyl-1-cyclohexyl)-3-hexanol revêt un intérêt particulier en raison de sa note olfactive ambrée et animale tout à la fois élégante et puissante. L'art antérieur fait état de deux procédés pour sa préparation [voir brevet cité plus haut]. L'un a recours à la 2,3,6-triméthyl-cyclohex-5-ène-1-one en tant que produit de départ selon le schéma que voici : 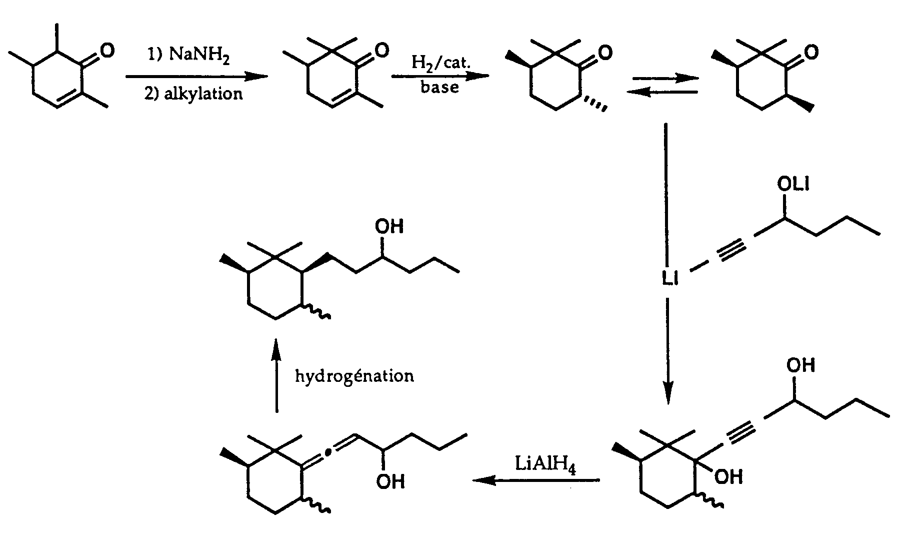
L'autre procédé emploie par contre des dérivés esters de l'acide alpha-cyclogéranique et peut être illustré comme suit : 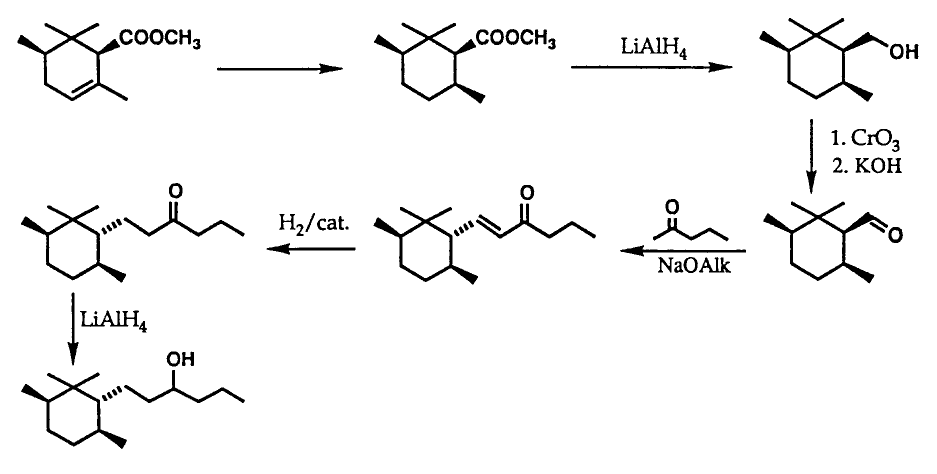
Comme il apparait à l'examen du schéma ci-dessus, l'un des composés critiques dans ce procédé est constitué par le 2,2,3,6-tétraméthyl-cyclohexane-carbaldéhyde.

La présente invention apporte une solution nouvelle et techniquement satisfaisante au problème posé par la préparation de cet aldéhyde, notamment du composé dont la configuration est définie par la formule suivante 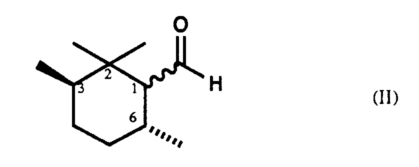
dans laquelle la ligne ondulée sert à désigner une liaison C-C de configuration cis ou trans, et dans laquelle les deux substituants méthyle en positions 3 et 6 possèdent une configuration trans. Cette solution passe par la préparation d'un époxyde nouveau ou 4,4,5,8-tétraméthyl-1-oxaspiro[2.5]octane, lequel constitue l'un des objets de l'invention. Grâce au procédé décrit plus bas il est désormais possible de préparer le 4,4,5,8-tétraméthyl-1-oxaspiro[2.5]octane dans la forme isomérique particulière comportant les deux substituants méthyle en positions 5 et 8 dans la configuration trans 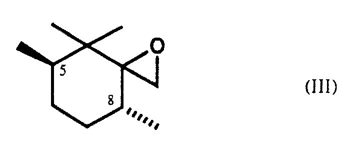
ce qui permet l'obtention, par sa transformation suivante, du 1-(2,2,c3,t6-tétraméthyl-1-cyclohexyl)-3-hexanol de formule 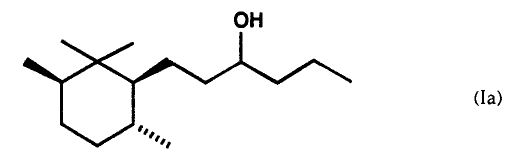
dont l'emploi en tant qu'agent parfumant a été décrit plus haut.

La présente invention a également pour objet un procédé pour la préparation du 4,4,5,8-tétraméthyl-1-oxaspiro[2.5]octane, lequel procédé consiste en la réaction de la 2,2,3,6-tétraméthyl-cyclohexanone avec le méthylure de diméthylsulfonium. On connait de l'art antérieur des procédés de ce type, lesquels s'effectuent dans les conditions réactionnelles décrites par E.J. Corey et M. Chaykovsky [J. Am. Chem. Soc 87, 1353 (1965)] pour des transformations analogues. Le méthylure de diméthylsulfonium peut par exemple être préparé in situ par la réaction entre l'hydrure de sodium, le diméthylsulfure et le sulfate de diméthyle en solution dans le diméthylsulfoxyde.

Selon une variante de ce procédé [voir, par exemple, K.H. Schulte-Elte et al., Helv. Chim. Acta 68, 1961 (1985)] le méthylure de diméthylsulfonium est obtenu par réaction d'un halogénure, notamment un chlorure ou un iodure, de triméthylsulfonium avec l'hydrure de sodium dans le diméthylsulfoxyde.

D'autre part, M. Rosenberger et al. [Helv. Chim. Acta 63, 1665 (1980)] avaient décrit la transformation de cétones insaturées telles que la β-ionone en les oxiranes correspondants, suivant la méthode suggérée par Corey-Chaykovsky [voir réf. citée], par réaction de ces cétones avec le chlorure de triméthylsulfonium, en présence de chlorure de triéthylbenzylammonium et à l'aide de NaOH aqueux. Ces auteurs avaient cependant constaté que la transformation analogue de la 2,2,6-triméthyl-5-cyclohexén-1-one nécessitait un temps de réaction particulièrement long (10 jours).

Un autre procédé de ce type est encore connu de la demande de brevet européen N^{o} 124 011 qui décrit la préparation d'époxydes connus, utiles pour l'obtention de fongicides et de régulateurs de la croissance de plantes. Ces époxydes étaient préparés par réaction des cétones appropriées avec le méthylure de diméthylsulfonium, ce dernier étant obtenu in situ par la réaction de diméthylsulfure et diméthylsulfate avec l'hydroxyde de potassium ou de sodium utilisé sous forme de poudre.

La présente invention a pour objet un procédé pour la préparation de 4,4,5,8-tétraméthyl-1-oxaspiro[2.5]octane, par traitement de la 2,2,3,6-tétraméthyl-1-cyclohexanone avec le méthylure de diméthylsulfonium dans les conditions de la réaction dite de Corey-Chaykovski, le méthylure de diméthylsulfonium étant obtenu par réaction d'un sel soufré approprié avec l'hydroxyde de sodium dans un solvant organique, le procédé étant caractérisé en ce que l'hydroxyde de sodium est utilisé sous forme de pastilles solides et en large excès par rapport aux sels souffrés utilisés comme produits de départ.

Dans les conditions de réaction définies dans le procédé de l'invention, nous avons observé que la conversion en l'oxirane désiré pouvait être complétée en six heures environ lorsque le méthylure de diméthylsulfonium était obtenu à partir de chlorure de triméthylsulfonium dans le diméthylsulfoxyde. Des temps de réaction plus longs, de l'ordre de 18 à 40 h environ ont été par contre observés lorsque le méthylure de diméthylsulfonium a été préparé in situ dans le milieu réactionnel à partir de diméthylsulfure et diméthylsulfate avec l'hydroxyde de sodium dans le diméthylsulfoxyde.

La 2,2,3,6-tétraméthyl-1-cyclohexanone, sous sa forme isomérique trans, utilisée comme produit de départ dans le procédé décrit ci-dessus, peut être préparée conformément à la méthode décrite par G. Schäppi et C.F. Seidel [Helv. Chim. Acta, 30, 2199 (1947)].

Le procédé s'effectue à une température comprise entre environ 10 et 50°C, de préférence au voisinage de la température ambiante.

Selon un mode d'exécution particulier, le NaOH est ajouté à un mélange constitué par la 2,2,3,6-tétraméthyl-1-cyclohexanone et le chlorure de triméthylsulfonium dans le diméthylsulfoxyde. La réaction est légèrement exothermique, d'où la nécessité de procéder à un refroidissement extérieur du mélange réactionnel de manière à maintenir la température à environ 25-30°C. Le mélange est maintenu sous bonne agitation pendant 6 heures environ. Le 4,4,5,8-tétraméthyl-1-oxaspiro[2.5]octane est ensuite obtenu par extraction du mélange résultant à l'éther de pétrole, concentration des extraits organiques et distillation fractionnée.

L'oxirane obtenu peut être transformé en 2,2,3,6-tétraméthyl-cyclohexane-carbaldéhyde par réaction avec un acide de Lewis tel le trifluoroboroéthérate, le tétrachlorure de titane ou le tétrachlorure d'étain. La réaction peut s'effectuer également à l'aide d'autres réactifs tels des halogénures métalliques comme le ZnI₂, le MgBr₂ ou le MgI₂. La transformation en l'aldéhyde désiré peut aussi avoir lieu en soumettant ledit oxirane à pyrolyse à une température comprise entre environ 400 et 500°.

Par le procédé de l'invention, et en utilisant la 2,2,3,6-tétraméthyl-1-cyclohexanone sous forme d'un mélange isomérique trans/cis (82 : 18), on obtient le 4,4,5,8-tétraméthyl-1-oxaspiro[2.5]octane sous forme d'un mélange isomérique dont le contenu en l'isomère trans est prépondérant, de l'ordre de 95%, ce qui rend le produit ainsi obtenu particulièrement attrayant en tant que produit de départ pour la préparation du 1-(2,2,3,6-tétraméthyl-1-cyclohexyl)-3-hexanol de qualité olfactive supérieure.

Compte tenu de la présence d'un noyau oxiranique dans sa molécule, le 4,4,5,8-tétraméthyl-1-oxaspiro[2.5]octane peut également se présenter sous l'une des formes isomériques définies à l'aide des formules suivantes : 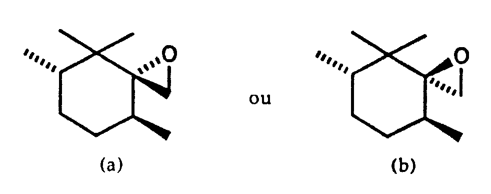
Le contenu réciproque de l'un ou l'autre de ces isomères peut varier légèrement en fonction des conditions dans lesquelles la réaction est effectuée, sans pour autant influencer le déroulement de la transformation du 4,4,5,8-tétraméthyl-1-oxaspiro[2.5]octane en 1-(2,2,3,6-tétraméthyl-1-cyclohexyl)-3-hexanol.

Les deux diastéréoisomères à l'état pur peuvent également être obtenus à partir de la 2,2,t-3,r-6-tétraméthyl-1-cyclohexanone optiquement active par un procédé analogue à celui qui a été décrit plus haut.

C'est ainsi qu'à partir de la (+)-(3S, 6S)-2,2,3,6-tétraméthyl-1-cyclohexanone, ayant [alpha]²⁰D = + 52,2°, on obtient le (+)-(3S, 5S, 8S)-4,4,5,8-tétraméthyl-1-oxaspiro[2.5]octane ayant [alpha]²⁰D = + 36,0°.

A partir de la (-)-(3R, 6R)-2,2,3,6-tétraméthyl-1-cyclohexanone, ayant [alpha]²⁰D = - 50,7°, on obtient le (-)-(3R, 5R, 8R)-4,4,5,8-tétraméthyl-1-oxaspiro [2.5]octane, dont le [alpha]²⁰D est égal à -35,1°.

Quoique l'aldéhyde de formule 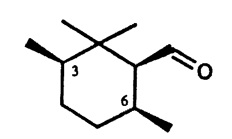
ait été décrit dans le brevet européen n^{o} 121'828 ainsi que dans Helv. Chim. Acta, 68, 1961 (1985), son isomère de formule (II), comportant les deux substituants méthyle en positions 3 et 6 de configuration trans, est un composé nouveau. Il constitue également un des objets de la présente invention. Il peut se présenter sous l'une des formes diastéréomères suivantes : 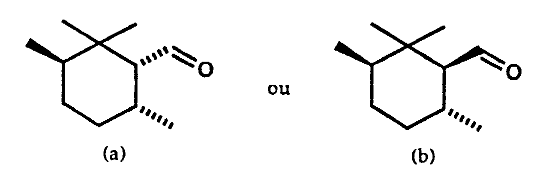
La présente invention est illustrée de manière plus détaillée par les exemples suivants dans lesquels les températures sont indiquées en degrés centigrades et les abréviations ont le sens usuel dans l'art.

### Exemple 1

### Préparation de 4,4,t-5,r-8-tétraméthyl-1-oxaspiro[2.5]octane

A. A partir du chlorure de triméthylsulfonium
   Dans un réacteur de 1 l muni de réfrigérant et d'un agitateur, on a placé 150 g (1,33 M) de chlorure de triméthylsulfonium et 156 g (0,95 M, pureté 94%) de 2,2,t-3,r-6-tétraméthyl-1-cyclohexanone dans 500 ml de diméthylsulfoxyde anhydre. A ce mélange on a ajouté 240 g (6 M) de NaOH en pastilles tout en agitant vigoureusement. Après 15 min la température du mélange réactionnel avait atteint 30° et cette valeur a été maintenue en procédant à un refroidissement extérieur. Le déroulement de la réaction a été suivi par contrôle GC. La réaction complète s'effectue en 6 h 15 min.
   Le mélange a été repris avec 200 ml d'éther de pétrole et le tout a été versé doucement dans un mélange de 2 l de glace/eau. Les phases organiques ont été séparées et ajoutées à une fraction de 100 ml d'éther de pétrole ayant servi au lavage de la phase aqueuse. Leur concentration a fourni un résidu qui, par distillation fractionnée sur une colonne de 20 cm comportant un remplissage de spirales, a fourni une fraction de 158 g ayant Eb. 41-43°/0,8 mmHg de l'oxirane désiré dont la pureté est de 95% (rend. 94%).
B. A partir d'un mélange de sulfure de diméthyle et de sulfate de diméthyle
   Dans un réacteur de 2 l on a placé un mélage constitué par 1,3 l de diméthylsulfoxyde, 185 g (3 M) de diméthylsulfure et 340 g (2,7 M) de diméthylsulfate. Ce mélange a été maintenu sous agitation douce pendant que sa température monte lentement jusqu'à 50°. La température a été maintenue à cette valeur par un refroidissement extérieur. Lorsque la température interne redescend d'elle-même on a cessé le refroidissement jusqu'à ce qu'elle atteigne la température ambiante. L'hydroxyde de sodium en pastilles a été alors ajouté [660 g (16,5 M)] au mélange, suivi de 350 g (2,3 M) de 2,2,t-3,r-6-tétraméthyl-1-cyclohexanone et le tout a été maintenu sous agitation pendant 18-40h.
   100 ml d'acétate d'éthyle ont été ajoutés, suivis, lentement, de 400 ml d'eau. La phase aqueuse séparée a été extraite successivement avec 100 ml d'éther de pétrole (30-50°), 100 ml d'acétate d'éthyle et de nouveau avec 100 ml d'éther de pétrole (30-50°). Les phases organiques réunies ont été lavées avec 2 fractions de 100 ml chacune d'une solution aqueuse saturée de NaCl, puis avec de l'eau. Après séchage sur Na₂SO₄, filtration et évaporation on a obtenu 355 g de l'oxirane désiré ayant une pureté de 92% (rend. 87%). Les caractères analytiques du produit obtenu sont les suivants :
   ¹H-RMN (360MHz) : 0,695(3H, d, J=3) ; 0,75(3H, s) ; 0,85(3H, d, J=7) ; 0,93 et 0,945(3H, 2s) ; 2,59-2,75(2H, m) δ ppm ;
   SM : M⁺ = 168(11) ; m/z : 153(66), 139(13), 123(91), 111(31), 107(11), 96(48), 81(100), 67(41), 55(68), 41(63).

### Exemple 2

### Préparation de 2,2,c-3,t-6-tétraméthyl-r-1-cyclohexane-carbaldéhyde

10 g (59,5 mM) de l'époxyde obtenu selon le procédé décrit dans l'exemple précédent dans 200 ml de toluène ont été brassés avec 2 ml de trifluoroboroéthérate sous atmosphère d'azote et à température ambiante pendant 15 min. Le mélange de réaction a été ensuite versé sur de la glace, lavé avec une solution aqueuse concentrée de NaCl, et la phase organique a été séparée. Par les traitements usuels de séchage sur Na₂SO₄, filtration et concentration à pression réduite, on a obtenu après épimérisation avec NaOMe/MeOH environ 10 g de l'aldéhyde désiré dont les données spectrales étaient les suivantes :
IR : 1710 cm⁻¹ ;
¹H-RMN (360MHz) : 0,805(3H, d, J=6,4) ; 0,83(3H, d, J=6,4) ; 0,91(3H, s) ; 0,96(3H, s) ; 1,01(1H, ddd, J=4 ; 12,6 ; 25,2 ); 1,15-1,24(1H, m) ; 1,36(1H, ddd, J=4 ; 12,6 ; 25,2); 1,41-1,5(1H, m) ; 1,6(1H, dd, J=5,4 ; 10,8) ; 1,73-1,82(1H, m) ; 1,9-2,08(1H, m) ; 9,67(1H, d, J=5,4) δ ppm;
SM : 168(M⁺, 9) ; m/z : 161(0), 145(0), 139(2), 135(8), 124(21), 109(11), 98(42), 83(100), 69(62), 55(85), 41(44).

### Exemple 3

### Préparation de (+)-(3S, 5S, 8S)-4,4,5,8-tétraméthyl-1-oxaspiro[2.5]octane et (-)-(3R, 5R, 8R)-4,4,5,8-tétraméthyl-1-oxaspiro[2.5]octane

En suivant le procédé décrit à l'exemple 1, mais en utilisant comme produit de départ le (+)-(3S, 6S)-2,2,3,6-tétraméthyl-1-cyclohexanone, ayant [alpha]²⁰D = + 52,2° on a obtenu le produit désiré avec [alpha]²⁰D = + 36,0°. En utilisant le (-)-(3R, 6R)-2,2,3,6-tétraméthyl-1-cyclohexanone avec [alpha]²⁰D = - 50,7°, l'on obtient le (-)-(3R, 5R, 8R)-4,4,5,8-tétraméthyl-1-oxaspiro [2.5]octane : [alpha]²⁰D = - 35,1°.
La rotation spécifique a été mesurée sur des échantillons purs.

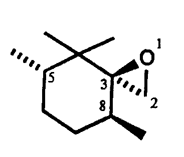

### 3,8-cis, 5,8-trans

¹H-RMN (360 MHz) : 0,695(3H, d, J=7) ; 0,75(3H, s) ; 0,84(3H, d, J=7) ; 0,93(3H, s) ; 2,65(2H, J=4) delta ppm ;
¹³C-RMN : 15,25(q) ; 16,42(q) ; 18,98(q) ; 20,82(q) ; 30,04(d) ; 31,31(t) ; 32,76(t) ; 37,95(s) ; 38,39(d) ; 45,84(t) ; 65,22(s) delta ppm ;
SM : M⁺ = 168; m/z : 153(90), 139(19), 123(91), 111(31), 107(15), 95(42), 81(95), 67(36), 55(68), 41(100).

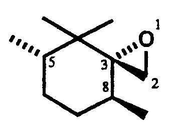

### 3,8-trans, 5,8-trans

¹H-RMN (360 MHz) : 0,695(3H, d, J=7) ; 0,75(3H, s) ; 0,86(3H, d, J=7) ; 0,94(3H, s) ; 2,62(2H, J=5) delta ppm ;
¹³C-RMN : 15,1(q) ; 16,0(q) ; 17,0(q) ; 22,4(q) ; 31,0(t) ; 31,1(d) ; 34,4(t) ; 38,1(s) ; 42,2(d) ; 45,2(t) ; 66,1(s) delta ppm ;
SM : M⁺ = 168 ; m/z : 153(90), 139(19), 123(91), 111(31), 107(15), 95(42), 81(95), 67(36), 55(68), 41(100).

En transformant les deux époxydes ainsi obtenus selon le procédé décrit à l'Exemple 5, on a obtenu les aldéhydes correspondants dont les caractéristiques analytiques étaient les suivantes : 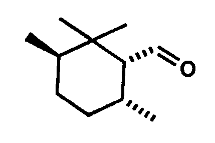
IR : 1710 cm⁻¹ ;
¹H-RMN (360 MHz) : 0,88(3H, d, J=7) ; 0,89(3H, d, J=7) ; 0,897(3H, s) ; 0,945(3H, s) ; 1,76-1,93(1H, m) ; 1,93(1H, t, J=6) ; 1,93-2,06(1H, m) ; 10,015(1H, d, J=6) delta ppm ;
SM : M⁺ = 168 ; m/z : 153(1), 135(6), 124(6), 109(8), 97(16), 91(4), 84(100), 69(30), 55(48), 41(27). 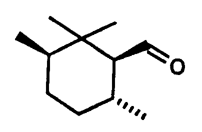
IR : 1710 cm⁻¹ ;
¹H-RMN (360 MHz) : 0,805(3H, d, J=6,4) ; 0,83(3H, d, J=6,4) ; 0,91(3H, s) ; 0,96(3H, s) ; 1,01(1H, J=4 ; 12,6 ; 25,2) ; 1,15-1,24(1H, m) ; 1,36(1H, ddd, J=4 ; 12,6 ; 25,2) ; 1,41-1,5(1H, m) ; 1,6(1H, dd, J=5,4 ; 10,8) ; 1,73-1,82(1H, m) ; 1,9-2,08(1H, m) ; 9,67(1H, d, J=5,4) delta ppm ;
¹³C-RMN : 15(q) ; 15,2(q) ; 20,7(q) ; 27,7(q) ; 28,1(d) ; 30,6(t) ; 34,7(t) ; 36,7(s) ; 41,8(d) ; 67,6(d) ; 207,7(d) delta ppm ;
SM : M⁺ = 168; m/z : 161(0), 145(0), 139(2), 135(8), 124(21), 109(11), 98(42), 83(100), 69(62), 55(85), 41(44).

Les deux enantiomères de la 2,2,3,6-tétraméthyl-1-cyclohexanone, utilisés comme produits de départ dans le procédé indiqué ci-dessus, peuvent être obtenus à partir du (-)-(S)-bêta-citronellol et de la (+)-(R)-pulégone, respectivement. Les procédés suivis sont illustrés à l'aide des schémas réactionnels suivants :

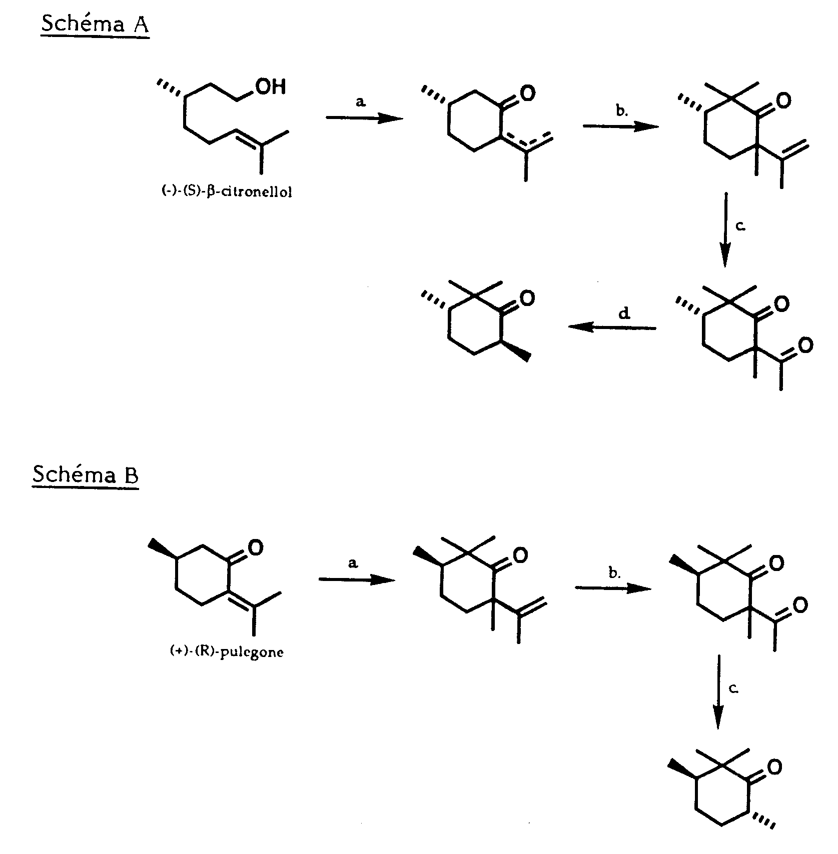

### A. Préparation de (+)-(3S, 6S)-2,2,3,6-tétraméthyl-1-cyclohexanone

a. Dans un réacteur 4 cols, munis d'agitation mécanique, d'un thermomètre, d'un réfrigérant et d'un tube d'introduction d'azote on a chargé 10 l de CH₂Cl₂ et 1704 g (7,89 M) de chlorochromate de pyridinium. A ce mélange on a ajouté goutte à goutte 400 g (2,56 M) de (-)-(S)-bêta-citronellol tout en maintenant la température à 15-20°, puis on a laissé sous agitation pendant environ 60 h à température ambiante. Après filtration, on a extrait avec du CH₂Cl₂ et les extraits organiques réunis ont été lavés avec HCl 15%, NaHCO₃ sat., puis avec de l'eau jusqu'à neutralité. Par évaporation et distillation sur résidu à 7,8 mmHg on a isolé 206,9 g de (-)-pulégone et (-)-isopulégone (40:60) (rend. 37%). Eb. 62°/7,8 mmHg.
b. 50 g (1,25 M) de KH à 20% dans l'huile ont été introduit à la fois dans un ballon contenant 1000 ml de tétrahydrofuranne anhydre, puis on a ajouté goutte à goutte au mélange résultant 51 g (0,34 M) de (-)-pulégone. La réaction est légèrement exothermique et elle a été complétée par un réchauffement à reflux pendant 2 h. Après avoir laissé redescendre la température à 45° environ, on a remplacé le réfrigérant ordinaire par un réfrigérant à CO₂/acétone et on a introduit goutte à goutte dans le mélange, 78 ml (1,25 M) de CH₃I. On a brassé à température ambiante pendant 1 heure puis on a ajouté prudemment au mélange 85 ml d'eau et le tout a été ensuite versé sur de la glace. Par extraction à l'éther, lavage avec de l'eau, séchage et concentration on a obtenu 213 g de résidu qui, par distillation sur résidu ont fourni 58 g de (+)-(3S)-6-isopropyl-2,2,3,6-tétraméthyl-1-cyclohexanone ; Eb. 82°/7,8 mmHg.
c. Un mélange de 238,7 g de la cétone obtenue sous lettre b. ci-dessus à 52% (soit 124,8 g de cétone pure ; 0,6 M) et 620 ml d'acétate d'éthyle a été refroidi à - 10° tandis qu'un courant d'ozone a été introduit dans le mélange durant 6 h, jusqu'à la fin de l'exothermie, puis 240 ml de diméthylsulfure ont été introduit goutte à goutte sous azote à -10°.
   Après avoir laissé remonter la température à la valeur ambiante, le mélange a été brassé pendant 1 nuit. Après concentration, on a repris à l'éther et on a soumis les extraits organiques aux traitements usuels pour fournir 242,1 g de résidu. La (+)-(3S)-6-acétyl-2,2,3,6-tétraméthyl-1-cyclohexanone a été obtenue à Eb. 76°/6,2 mmHg (rend. 44%).
d. 148,8 g de la cétone obtenue sous lettre c. ci-dessus (pureté 35% ; soit 52,08 g de cétone pure ; 0,27 M) en mélange avec 114,7 g (2,05 M) de KOH en pastilles et 4000 ml d'éthanol ont été chauffés à reflux durant 2 h. Le mélange a été concentré et extrait ensuite à l'éther de pétrole (30-50°). Les extraits combinés ont été soumis aux traitements usuels et ont fourni par évaporation 95,6 g de résidu. Par distillation fractionnée avec une colonne Fischer on a isolé 27,1 g de la cétone désiré ayant Eb. 56°/5,8 mmHg.

### B. Préparation de (-)-(3R, 6R)-2,2,3,6-tétraméthyl-1-cyclohexanone

89,1 g (2,23 M) de KH à 35% dans l'huile ont été verse à la fois dans un réacteur contenant 2000 ml de tétrahydrofuranne anhydre, puis on a ajouté goutte à goutte au mélange résultant 96,7 g (0,636 M) de (+)-pulégone à 15-20°. On a ensuite procédé comme indiqué sous lettre A.b. ci-dessus en utilisant 139 ml (2,23 M) de CH₃I. On a ainsi obtenu 64,3 g (rend. 52%) de (-)-(3R)-6-isopropényl-2,2,3,6-tétraméthyl-1-cyclohexanone.
Le produit ainsi obtenu a été ensuite traité de manière analogue à ce qui a été indiqué aux lettres A.c. et A.d. pour fournir la (-)-(3R, 6R)-2,2,3,6-tétraméthyl-1-cyclohexanone.

## Revendications

1. 4,4,5,8-Tétraméthyl-1-oxaspiro[2.5]octane.

2. Composé selon la revendication 1 sous l'une des formes diastéréomères de formule

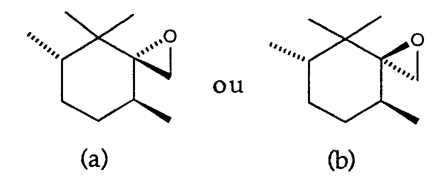

3. Procédé pour la préparation de 4,4,5,8-tétraméthyl-1-oxaspiro[2.5] octane, par traitement de la 2,2,3,6-tétraméthyl-1-cyclohexanone avec le méthylure de diméthylsulfonium dans les conditions de la réaction dite de Corey-Chaykovski, le méthylure de diméthylsulfonium étant obtenu par réaction d'un sel soufré approprié avec l'hydroxyde de sodium dans un solvant organique, le procédé étant caractérisé en ce que l'hydroxyde de sodium est employé sous forme de pastilles solides et en large excès par rapport au sel soufré utilisé comme réactif de départ.

4. Procédé selon la revendication 3, caractérisé en ce que l'hydroxyde de sodium est utilisé en un excès de l'ordre de 5 fois le poids de sel soufré.

5. Procédé selon la revendication 3 ou 4, caractérisé en ce que le solvant organique est le diméthylsulfoxyde.

6. Procédé selon l'une des revendications 3 à 5, caractérisé en ce qu'on utilise comme produit de départ la 2,2,3,6-tétraméthyl-2-cyclohexanone éminemment sous sa forme isomérique de formule 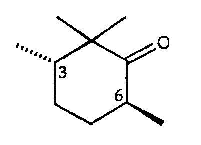 et qu'on obtient le 4,4,5,8-tétraméthyl-1-oxaspiro[2.5]octane éminemment sous ses formes isomériques telles que définies à la revendication 2.

7. Utilisation du 4,4,5,8-tétraméthyl-1-oxaspiro[2.5]octane à titre de produit de départ pour la préparation du 2,2,3,6-tétraméthyl-cyclohexane-carbaldéhyde, caractérisée en ce qu'on le traite avec un acide de Lewis.

8. 2,2,3,6-Tétraméthyl-cyclohexane-carbaldéhyde sous l'une des formes diastéréomères de formule

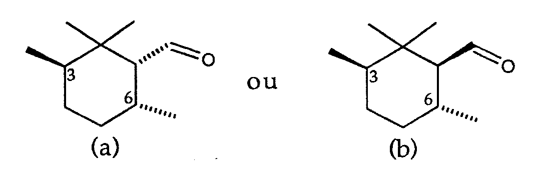

## Claims

1. 4,4,5,8-Tetramethyl-1-oxaspiro[2.5]octane.

2. Compound according to claim 1 in one of the diastereomeric forms of formula

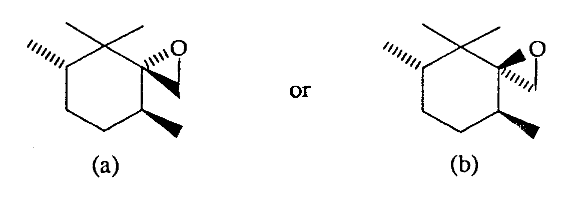

3. Process for the preparation of 4,4,5,8-tetramethyl-1-oxaspiro[2.5]octane, by treatment of 2,2,3,6-tetramethyl-1-cyclohexanone with dimethylsulfonium methylide under the conditions of the so-called Corey-Chaykovsky reaction, said dimethylsulfonium methylide being obtained by reaction of an appropriate sulphur salt with sodium hydroxide in an organic solvent, the process being characterized in that said sodium hydroxide is used in the form of solid pellets and in large excess relative to the sulphur salt used as starting product.

4. Process according to claim 3, characterized in that the sodium hydroxide is used in an excess of the order of 5 times the weight of sulphur salt.

5. Process according to claim 3 or 4, characterized in that the organic solvent is dimethylsulfoxide.

6. Process according to any one of claims 3 to 5, characterized in that one uses as starting product 2,2,3,6-tetramethyl-1-cyclohexanone essentially in its isomeric form of formula 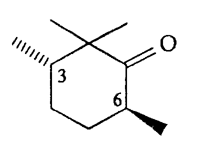 and in that 4,4,5,8-tetramethyl-1-oxaspiro[2.5]octane is obtained essentially in one of its isomeric forms such as defined in claim 2.

7. Utilization of 4,4,5,8-tetramethyl-1-oxaspiro[2.5]octane as a starting product for the preparation of 2,2,3,6-tetramethyl-cyclohexane-carbaldehyde characterized in that it is treated with a Lewis type acid.

8. 2,2,3,6-Tetramethyl-cyclohexane-carbaldehyde in one of a diastereomeric forms of formula

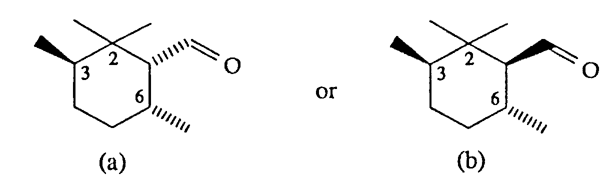

## Patentansprüche

1. 4,4,5,8-Tetramethyl-1-oxaspiro[2.5]octan.

2. Verbindung gemäß Anspruch 1, in einer der diastereomeren Formen der Formel

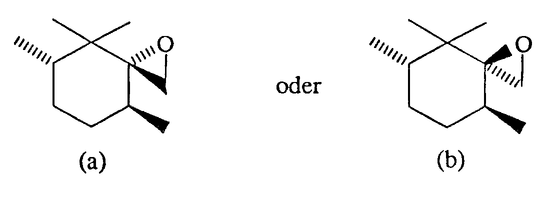

3. Verfahren zur Herstellung voll 4,4,5,8-Tetramethyl-1-oxaspiro [2.5]octan, durch Behandlung von 2,2,3,6-Tetramethyl-1-cyclohexanon mit Dimethylsulfoniummethylid unter den Corey-Chaykovsky Reaktionsbedingungen, wobei das Dimethylsulfoniummethylid durch Reaktion eines geeigneten Schwefelsalzes mit Natriumhydroxyd in einem organischen Lösungsmittel erhalten wird, dadurch gekennzeichnet, daß man das Natriumhydroxyd in der Form von festen Tabletten und in große m Überschuß, verglichen zum als Ausgangsprodukt verwendeten Schwefelsalzes, verwendet.

4. Verfahren gemäß Anspruch 3, dadurch gekennzeichnet, daß das Natriumhydroxyd in fünffachen Gewichtsüberschuß des Schwefelsalzes verwendet wird.

5. Verfahren gemäß Anspruch 3 oder 4, dadurch gekennzeichnet, daß das organische Lösungsmittel Dimethylsulfoxyd ist.

6. Verfahren gemäß einem der Ansprüche 3 bis 5, dadurch gekennzeichnet, daß man als Ausgangsprodukt 2,2,3,6-Tetramethyl-1-cyclohexanon im wesentlichen in der isomerischen Form der Formel 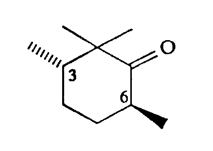 verwendet, um 4,4,5,8-Tetramethyl-1-oxaspiro[2.5]octan im wesentlichen in einer der isomerischen Formen gemäß Anspruch 2 zu erhalten.

7. Verwendung von 4,4,5,8-Tetramethyl-1-oxaspiro [2.5]octan als Ausgangsprodukt zur Herstellung von 2,2,3,6-Tetramethyl-cyclohexan-carbaldehyd, dadurch gekennzeichnet, daß man es mit einer Lewissäure behandelt.

8. 2,2,3,6-Tetramethyl-cyclohexan-carbaldehyd in einer der diastereomeren Formen der Formel
